Europäisches Patentamt

European Patent Office

Office européen des brevets

(19)

(11) Publication number : **0 401 894 B1**

## (12) EUROPEAN PATENT SPECIFICATION

(45) Date of publication of patent specification :
**28.10.92 Bulletin 92/44**

(51) Int. Cl.$^5$ : **A61K 31/675, A61K 9/14**

(21) Application number : **90201352.3**

(22) Date of filing : **28.05.90**

(54) **Freeze-dried, powdery cyclophosphamide composition, as well as process for its preparation.**

(30) Priority : **06.06.89 NL 8901431**

(43) Date of publication of application :
**12.12.90 Bulletin 90/50**

(45) Publication of the grant of the patent :
**28.10.92 Bulletin 92/44**

(84) Designated Contracting States :
**AT BE CH DE DK ES FR GB GR IT LI LU NL SE**

(56) References cited :
**EP-A- 0 271 622**
**US-A- 4 537 883**
**BUNDESVERBAND DER PHARMAZEUTIS-
CHER INDUSTRIE: "Rote liste 1989", Editio
Cantor, Aulendorf, Württ, DE**

(73) Proprietor : **PHARMACHEMIE B.V.
Swensweg 5
NL-2031 GA Haarlem (NL)**

(72) Inventor : **Nijkerk, Alfred James
Nicolaas Witsenkade 17
NL-1017 ZS Amsterdam (NL)**
Inventor : **Vermeer, Johanna Maria Pieternella
Veldhorststraat 11
NL-2161 EP Lisse (NL)**

(74) Representative : **Ellowicz, Leo, Drs. et al
Octrooibureau Polak & Charlouis Laan Copes
van Cattenburch 80
NL-2585 GD Den Haag (NL)**

## Description

The invention relates to a freeze-dried, powdery pharmaceutically acceptable cyclophosphamide and to a process for preparing same.

Freeze-dried powdery cyclophosphamide compositions are known in several forms, as appears e.g. from Journal of Parenteral Science and Technology, Vol. 42, no. 1, pages 29-37 and no 5, pages 165-173. According to the abovementioned articles, cyclosphosphamide, mixed with a carrier was freeze-dried to a completely anhydrous product and then wetted, in most cases yielding the monohydrate. From the abovementioned articles it further appears that the stability of-in particular a mixture of cyclophosphamide with mannitol is substantially improved if the cyclophosmamide is converted to the monohydrate. According to the first mentioned article it was already known that cyclosphosphamide-monohydrate is crystaline and stable at room temperature, but quickly decomposes at elevated temperatures and in aqueous solution. Very little was known of the stability of freeze-dried cyclophosphamide. In the abovementioned articles no experiments with freeze-dried cyclophosphamide-monohydrate have been reported, but the monohydrate was prepared from the freeze-dried anhydrate and only tested for stability in solution.

In the past a pharmaceutical composition has been marketed which contains the cyclophosphamide-monohydrate in the form of a coarse powder, mixed with common salt for the purpose of making it isotonic, but in the meantime this composition has been replaced by the freeze-dried mixture of cyclophosphamide-monohydrate and mannitol.

Further references illustrating the above state of the art are US-A-4,537,883 and EP-A-271622.

The latter reference discloses on page 3, lines 14 a.f. that "CPA-hydrate, whether by itself, or with an excipient, is known to defy freeze-drying so as to give a pharmaceutically elegant product" by "pharmaceutically elegant product" a visually pleasing product is meant. Furthermore , it is stated in this reference on page 4, lines 35/36 that efforts to freeze-dry CPA-monohydrate without any addition were not successful.

"Rote Liste 1989" number 85006 relates to a composition of cyclosphosphamide-monohydrate in the form of dragees and powders with can be reconstituted for injection. There is no indication that this product would not have been prepared in accordance with the above principles, and it is mentioned that storage prescriptions should be given.

The compositions according to the above discussed state of the art also have the drawback that the cyclophosphamide is always delivered in a mixture with another substance.

It has now been found that freeze-dried, powdery pharmaceutically acceptable cyclophosphamide can be obtained in the form of "pure" cyclophosphamide-monohydrate without any further addition. ("Pure" referring to to a monohydrate without any hydrates being present). In addition to the above product, the invention provides a process for preparing a freeze-dried, powdery pharmaceutically acceptable cyclophosphamide, which is characterized by the fact that the cyclophosphamide is freeze-dried without any addition and the freeze-drying conditions are controlled in such a way that pure monohydrate is obtained.

The freeze-dried cyclophosphamide-monohydrate according to the invention is not only stable, but has the further advantage that it has a satisfactory appearance and dissolves quickly, while it does not contain any further addition.

Freeze-drying cyclophosphamide in such a way that pure monohydrate is obtained, per se does not offer particular difficulties. Of course, the correct conditions are dependent on the used freeze-drying apparatus. The following examples describe experiments with freeze-drying apparatus which are now used the applicant. However, it should be stressed here that when using other apparatus the optimum conditions thereof may be different.

## Example 1

Freeze-drying of 100 vials of cyclophosphamide, 500 mg

```
Freezing        4 hours  at   -70°C
Freeze-drying   20 hours at   plate temperature -8 to -4°C
                              condenser temperature -70°C
                              pressure 70-90 Pa
                38 hours at   plate temperature -2 to 0°C
                              condenser temperature -70°C
                              pressure 90-120 Pa
                10 hours at   plate temperature -10°C
                              condenser temperature -70°C
                              pressure 200-300 Pa
```

A perfect monohydrate was obtained.

## Example 2

Freeze-drying of 100 vials of cyclophosphamide, 100 mg

```
Freezing        4 hours  at   -20°C
                4 hours  at   -40°C
Freeze-drying   2 hours  at   plate temperature -2°C
                              condenser temperature -40°C
                              pressure 90-120 Pa
                20 hours at   plate temperature -10°C
                              condenser temperature -40°C
                              pressure 200-300 Pa
```

A perfect monohydrate was obtained.

## Example 3

Freeze-drying of 1200 vials of cyclophosphamide, 100 mg

```
Freezing        2 hours  at   -70°C
Freeze-drying   16 hours at   plate temperature -2 to 0°C
                              condenser temperature -70°C
                              pressure 100-200 Pa
                14 hours at   plate temperature -10°C
                              condenser temperature -70°C
                              pressure 200-300 Pa
```

A perfect monohydrate was obtained.

## Stability tests

The products of the above examples which were glass vials, containing the amounts of cyclophosphamide-monohydrate mentioned in the examples were stored at a temperature of 22-25°C. The results are indicated in the following tables. In these tables n.c. means (not changed).

Table 1

Product of example 1

|  | Initial | After | | | | | | |
|---|---|---|---|---|---|---|---|---|
|  |  | 2 | 3 | 6 | 9 | 12 | 18 months |
| Appearance | white cake | n.c. | n.c. | n.c. | n.c. | n.c. | n.c. |
| Water | 6.5% | — | — | 6.1% | 6.2% | 6.2% | 6.6% |
| Completeness solution | clear | n.c. | n.c. | n.c. | n.c. | n.c. | n.c. |
| pH | 4.4 | 4.4 | 4.0 | 4.3 | 3.7 | 4.0 | 3.7 |
| Assay Cyclophosph. o aq. | 97.3% | 98.7% | 96.7% | 97.7% | 96.8% | 97.4% | 98.4% |

## Table 2

EP 0 401 894 B1

Product of example 2

| | Initial | After | 2 | 3 | 6 | 9 | 12 | 18 | months |
|---|---|---|---|---|---|---|---|---|---|
| Appearance | white cake | | n.c. | n.c. | n.c. | n.c. | n.c. | n.c. | |
| Water | 6.3% | | — | — | 6.0% | 6.2% | 6.1% | 6.3% | |
| Completeness solution | clear | | n.c. | n.c. | n.c. | n.c. | n.c. | n.c. | |
| pH | 4.2 | | 4.3 | 4.0 | 4.2 | 3.8 | 4.0 | 3.7 | |
| Assay Cyclophosph. o aq. | 96.6% | | 98.2% | 96.4% | 98.8% | 96.7% | 96.1% | 97.0% | |

EP 0 401 894 B1

## Table 3

Product of example 3

| | Initial | After | 3 | 6 | 9 | 12 | months | 24 | 36 | 48 | 60 |
|---|---|---|---|---|---|---|---|---|---|---|---|
| Appearance | white cake/ powder | | n.c. | n.c. | n.c. | n.c. | | | | | |
| Water | 7.3% | | 6.7% | 6.5% | 6.3% | 6.1% | | | | | |
| Completeness solution | clear colorless | | n.c. | n.c. | n.c. | n.c. | | | | | |
| pH | 4.1 | | 4.2 | 4.0 | 3.6 | 4.0 | | | | | |
| Assay Cyclophosph. o aq. | 101.4% | | 102.1% | 100.8% | 103.9% | 102.7% | | | | | |
| Sterility | sterile | | - | sterile | - | sterile | | | | | |

## Claims

### Claims for the following Contracting States : AT, BE, CH, DE, FR, GB, IT, LI, LU, NL, SE

1. Freeze-dried, powdery, pharmaceutically acceptable cyclosphosphamide, characterised by the fact that it is pure cyclosphosphamide-monohydrate without any further addition.

2. Process for preparing freeze-dried, powdery, pharmaceutically acceptable cyclophosphamide, characterized by the fact that cyclophosphamide is freeze-dried without any further addition and the freeze-drying conditions are controlled in such a way that pure monohydrate is obtained.

### Claims for the following Contracting States : ES, GR

1. Process for preparing freeze-dried, powdery pharmaceutically acceptable cyclophosphamide, characterized by the fact that cyclophosphamide is freeze-dried without any further addition and the freeze-drying conditions are controlled in such a way that pure monohydrate is obtained.

## Patentansprüche

### Patentansprüche für folgenden Verstragsstaaten : DE, GB, FR, IT, NL, SE, LI, CH, BE, AT, LU, DK

1. Lyophilisiertes, pulveriges, für pharmazeutische Zwecke geëignetes Cyclophosphamid, **dadurch gekennzeichnet** daß es reines Cyclophosphamid ohne weiteren Zusatz ist.

2. Verfahren zur Herstellung eines lyophilisierten, pulverigen, für pharmazeutische Zwecke geëigneten Cyclophosphamids, **dadurch gekennzeichnet** daß man Cyclophosphamid ohne weiteren Zusatz lyophilisiert und die Verhältnisse so regelt, daß ein Monohydrat erhalten wird.

### Patentansprüch für folgende Vertragsstaaten : ES, GR

1. Verfahren zur Herstellung eines lyophilisierten, pulverigen, für pharmazeutische Zwecke geëigneten Cyclophosphamids, **dadurch gekennzeichnet** daß man Cyclophosphamid ohne weiteren Zusatz lyophilisiert und die Verhältnisse so regelt, daß ein Monohydrat erhalten wird.

## Revendications

### Revendications pour les Etats contractants suivants : DE, GB, IT, NL, SE, LI, CH, BE, AT, LU, DK

1. Cyclophosphamide lyophilisée pulvérulente, pharmaceutiquement acceptable, caractérisée en ce qu'il s'agit du monohydrate de cyclophosphamide pur, sans aucune addition complémentaire.

2. Procédé pour préparer la cyclophosphamide lyophilisée pulvérulente, pharmaceutiquement acceptable, caractérisé par le fait que la cyclophosphamide est lyophilisée, sans aucune addition complémentaire et que les conditions de lyophilisation sont réglées de manière que le monohydrate pur soit obtenu.

### Revendications pour les Etats contractants suivants : ES, GR

1. Procédé pour préparer la cyclophosphamide lyophilisée pulvérulente, pharmaceutiquement acceptable, caractérisé par le fait que la cyclophosphamide est lyophilisée, sans aucune addition complémentaire et que les conditions de lyophilisation sont réglées de manière que le monohydrate pur soit obtenu.